# EUROPEAN PATENT SPECIFICATION

(11) **EP 2 740 348 B1**
(45) Date of publication and mention of the grant of the patent: **06.05.2020**
(21) Application number: 12822404.5
(22) Date of filing: 03.08.2012
(51) Int. Cl.: A01G 7/04, A01G 33/00, A01H 3/02, C12N 1/12, C12N 13/00

(54) **PLANT CULTIVATION METHOD**
PFLANZENZUCHTVERFAHREN
PROCÉDÉ DE CULTURE DE PLANTES

(30) Priority: 05.08.2011 JP 2011172089; 27.03.2012 WO PCT/JP2012/057859
(43) Date of publication of application: 11.06.2014
(73) Proprietor: Showa Denko K.K., Tokyo 105-8518 (JP); Yamaguchi University, Yamaguchi 753-8511 (JP)
(72) Inventor: SHIGYO, Masayoshi, Yamaguchi-shi, Yamaguchi 753-8515 (JP); SUZUKI, Hiroshi, Tokyo 105-8518 (JP); YAMAUCHI, Naoki, Yamaguchi-shi, Yamaguchi 753-8515 (JP); ARA, Hironori, Tokyo 105-8518 (JP); SHIMOKAWA, Akihiro, Yamaguchi-shi, Yamaguchi 753-8515 (JP); MATSUMOTO, Misato, Yamaguchi-shi, Yamaguchi 753-8515 (JP); TONOOKA, Yuki, Ube-shi, Yamaguchi 755-8505 (JP)
(74) Representative: Strehl Schübel-Hopf & Partner
(86) International application number: PCT/JP2012/069884
(87) International publication number: WO 2013/021952

(56) References cited:
- WO-A1-2009/067194
- JP-A- 8 103 167
- JP-A- 2005 151 850
- JP-A- 2006 050 988
- JP-A- 2008 142 005
- US-A- 3 930 335
- US-A1- 2009 288 340
- R.M. FORSTER ET AL: "Influence of blue light on the photosynthetic capacity of marine plants from different taxonomic, ecological and morphological groups", EUROPEAN JOURNAL OF PHYCOLOGY, vol. 29, no. 1, 17 February 1994 (1994-02-17), pages 21-27, XP055576450, DE ISSN: 0967-0262, DOI: 10.1080/09670269400650441

## Description

### Technical Field

The present invention relates to a plant cultivation method. More particularly, it relates to a plant cultivation method for promoting a favorable growth by irradiating an artificial light to a plant and the like.

### Background Art

Conventionally, plant cultivation methods involve a technology for promoting a seedling growth by irradiating an artificial light to the seedling of a plant. By promoting the growth of the plant, the cultivation period can be shortened and the number of harvests in an identical place can be increased. Moreover, the quantity of the harvests can be increased even within the same cultivation period if the plant can be grown to a larger size.

As a plant cultivation method utilizing an artificial light irradiation, a plant irradiation equipment comprising an alternate irradiation of a green light and a white light to a plant is disclosed for example in Patent Document 1. This irradiation equipment establishes the daytime-to-nighttime variation by irradiating the green light of a wavelength of 500 to 570 nm and the white light of a wavelength of 300 to 800 nm alternately, thereby facilitating the translocation effect of the plant while aiming at growing the plant.

Also for example in Patent Document 2, a light source for plant cultivation which irradiates a light energy for culture, growth, cultivation, and tissue culture by simultaneous or alternate turning on of a light emitting diode emitting a blue light (400 to 480 nm) and a light emitting diode emitting a red light (620 to 700 nm) is disclosed. This light source for plant cultivation intends to cultivation the plant at a high energy efficiency by irradiating the lights only of the wavelengths in agreement with the chlorophyll's light absorption peaks (around 450 nm and around 660 nm).

In Patent Document 2, it is prescribed that the blue light and the red light may be irradiated simultaneously or irradiated alternately (see "Claim 1" in the relevant document). In Patent Document 2, however, it is just described, when comparing the irradiation only with the blue light, the irradiation only with the red light, and the simultaneous irradiation with the blue light and the red light, that, under the simultaneous irradiation, a healthy growth similar to that observed in a cultivation under a solar light (compared with a non-healthy growth such as succulent growth observed under a single light irradiation) was observed (see Paragraph [0011] in the relevant document), and no growth promoting effect under the alternate irradiation with the blue light and the red light was identified. Accordingly, Patent Document 2 contains substantially no disclosure of a plant cultivation method by an alternate irradiation with a blue light and a red light.

### Citation List

### Patent Literatures

[Patent Document 1] Japanese Unexamined Patent Application Publication No. H06-276858
[Patent Document 2] Japanese Unexamined Patent Application Publication No. H08-103167

US 3 930 335 A and JP 2006 050 988 A disclose a plant cultivation method comprising the steps of irradiating a red illuminative light and irradiating a blue illuminative light to a plant separately and independently of each other within a certain time period.

### Summary of Invention

### Technical Problem

For the purpose of increasing the productivity, a plant cultivation method by an artificial light irradiation which is more convenient, highly energy efficient and excellent in growth promoting effect is demanded. A major object of the present invention is to provide a plant cultivation method which fulfills such a demand.

### Solution to Problem

As a result of our intensive study on the plant growth promoting effect of an artificial light irradiation, we surprisingly discovered that an extremely remarkable effect can be obtained by a method as simple as an alternate irradiation of a red light and a blue light.

The present invention provides the following.
[1] A plant cultivation method for conducting a step of irradiating a red illuminative light to a plant and a step of irradiating a blue illuminative light to the plant separately and independently of each other, wherein the irradiation time of the step of irradiating the red illuminative light and the step of irradiating the blue illuminative light is 0.1 hour or more and less than 48 hours, the steps are conducted alternately and successively, and the light quantity ratio of the red illuminative light and the blue illuminative light is 3: 1 to more than 1:1, i.e. a higher quantity of red light than blue light, wherein the light quantity is the photosynthetic photon flux density expressed as µmol/m²s.
[2] The plant cultivation method according to [1], wherein the irradiation time is 3 hours or more and 24 hours or less.
[3]
[4] The plant cultivation method according to any one of [1] to [3], wherein the plant is a leaf vegetable, a fruit, or cereal.

In this plant cultivation method (Shigyo Method), the step for irradiating the red illuminative light and the step for irradiating the blue illuminative light are conducted alternately and successively. As used herein, the phrase "alternately and successively" means that the irradiation cycle consisting of the step for irradiating the red illuminative light and the step for irradiating the blue illuminative light is repeated at least 2 cycles or more.

This description also discloses a plant cultivation equipment comprising: a light irradiation part for irradiating a red illuminative light and a blue illuminative light to the plant; and a controlling part for controlling the light irradiation part to conduct a step for irradiating the red illuminative light to the plant and a step for irradiating the blue illuminative light to the plant separately and independently of each other within a certain time period.

In this plant cultivation equipment, the aforementioned controlling part allows the light quantities, wavelengths, and/or irradiation times of the red illuminative light and the blue illuminative light irradiated from the aforementioned light irradiation part to be kept at certain values or to be varied in certain patterns. It is preferable that the aforementioned light irradiation part comprises light emitting diodes which emit a red light or a blue light.

Furthermore, this description also discloses a plant cultivation equipment comprising: a first light irradiation part which irradiates a red illuminative light; a second light irradiation part which irradiates a blue illuminative light; and, a carrying means for moving a plant between a position irradiated with the illuminative light originated from the first light irradiation part and a position irradiated with the illuminative light originated from the second light irradiation part.

In the present invention, the "plant" includes useful ones among those belonging to seed plants, in which leaf vegetables, fruits, and cereals are at least included. Also in the present invention, it is understood that the "plant" widely includes ferns and mosses.

### Advantageous Effects of Invention

According to the present invention, a plant cultivation method using an artificial light irradiation which is convenient, highly energy efficient, and capable of being excellent in terms of a plant cultivation effect such as a growth promoting effect is provided.

### Brief Description of Drawings

Fig. 1 is a schematic view illustrating the procedure of the plant cultivation method according to the first embodiment of the present invention.
Fig. 2 is a schematic view illustrating the procedure of the plant cultivation method according to the second embodiment of the present invention.
Fig. 3 is a schematic view illustrating the procedure of the plant cultivation method according to the third embodiment of the present invention.
Fig. 4 is a schematic view illustrating the construction of the plant cultivation equipment according to the second embodiment.
Fig. 5 is a drawing-substituting photograph showing the results of the growth 7 days after germination in Test Example 1.
Fig. 6 is a drawing-substituting photograph showing the results of the growth 14 days after germination in Test Example 1.
Fig. 7 is a drawing-substituting photograph showing the results of the growth 21 days after germination in Test Example 1.

### Description of Embodiments

The preferred embodiments of the present invention are described below with referring to the drawings. The following embodiments are the examples of the representative of the embodiments of the present invention which do not serve to allow the scope of the invention to be interpreted narrowly. The description is made in the order shown below.
1. Plant cultivation method
   (1) Cultivation step
      (1-1) Plant cultivation method according to first embodiment
      (1-2) Plant cultivation method according to second embodiment
      (1-3) Plant cultivation method according to third embodiment
      (1-4) Wavelength
      (1-5) Light quantity (intensity)
      (1-6) Irradiation time
   (2) Condition establishing step
2. Plant cultivation equipment
   (1) Plant cultivation equipment according to first embodiment
      (1-1) Light irradiation part
      (1-2) Controlling part
   (2) Plant cultivation equipment according to second embodiment
3. Cultivated plants
   (1) Leaf vegetables
   (2) Fruits
   (3) Cereals
   (4) Mosses and the like

### 1. Plant cultivation method

### (1) Cultivation step

### (1-1) Plant cultivation method according to first embodiment

The plant cultivation method according to the present invention comprises a step for cultivating a plant by conducting a step for irradiating a red illuminative light to the plant (hereinafter referred to also as "red light irradiation step ") and a step for irradiating a blue illuminative light to the plant (hereinafter referred to also as "blue light irradiation step") separately and independently of each other within a certain time period.

The red illuminative light is an illuminative light containing a red light whose peak wavelength is 570 to 730 nm. The red illuminative light is acceptable just if containing the aforementioned red light, and may contain a light having a wavelength range different from that of the aforementioned red light, but preferably contains no blue light described below. The red illuminative light contains only the aforementioned red light in an especially preferred case. The blue illuminative light is an illuminative light containing a blue light whose peak wavelength is 400 to 515 nm. The blue illuminative light is acceptable just if containing the aforementioned blue light, and may contain a light having a wavelength range different from that of the aforementioned blue light, but preferably contains no red light described above. The blue illuminative light contains only the aforementioned blue light in an especially preferred case. Moreover, the red illuminative light contains no aforementioned blue light and the blue illuminative light contains no aforementioned red light in a preferred case, and the red illuminative light is exclusively the aforementioned red light and the blue illuminative light is exclusively the aforementioned blue light in an especially preferred case.

As used herein, "a certain time period" means a period of any length of the time during the plant cultivation. This period may be as long as the entire cultivation period. The shortest period may be set as desired as long as the effect of the invention can be exerted. This period may employ, for example an hour (h) as a time length unit, or may employ a longer time length unit (for example, day (D)) or a shorter time length unit (for example, minute (min)).

The plant cultivation method according to the present invention can be started or ended at any time during the entire period of the plant cultivation from the time immediately after the seed germination or immediately after planting the seedling through cropping, and can be applied over any time period.

The phrase "separately and independently of each other" means that the red light irradiation step and the blue light irradiation step are present separately during the aforementioned period. It is sufficient that at least each one step of the red light irradiation step and the blue light irradiation step is included in the aforementioned period, although 2 steps or more are included in a preferred case.

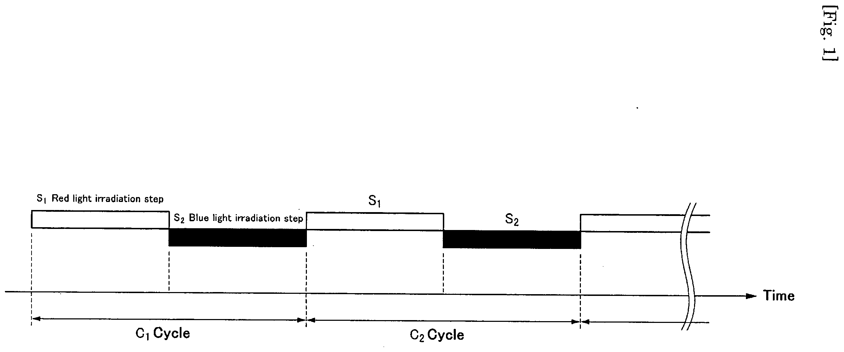

The red light irradiation step and the blue light irradiation step may be conducted alternately and successively, or may be conducted intermittently and repeatedly by allowing the both steps to be intervened by a step for irradiating the red illuminative light and the blue illuminative light simultaneously to the plant or by a step for interrupting the irradiation of the light to the plants. Nevertheless, it is preferable to conduct them alternately and successively for the purpose of enhancing the plant growing effect. These embodiments of the plant cultivation method according to the present invention are described in detail with referring to Fig. 1 to Fig. 3. It is also possible as a matter of course to conduct the plant cultivation method according to the present invention while combining the respective embodiments illustrated in Fig. 1 to Fig. 3 with each other as appropriate.

Fig. 1 is a schematic view illustrating the procedure of the plant cultivation method according to the first embodiment of the present invention. This embodiment conducts the red light irradiation step and the blue light irradiation step alternately and successively.

In the figure, the symbol S₁ represents the red light irradiation step, and the symbol S₂ represents the blue light irradiation step. In this embodiment, the red light irradiation step S₁ and the blue light irradiation step S₂ are conducted alternately and successively, and the irradiation cycle consisting of the red light irradiation step S₁ and the blue light irradiation step S₂ is conducted repeatedly.

By irradiating the red illuminative light and the blue illuminative light alternately to the plant as described above, the growth can markedly be promoted (see Examples described below). It is also possible to suppress succulent growth thereby increasing the crop.

While the case in which the procedure is started from the red light irradiation step S₁ in the first irradiation cycle C₁ is exemplified here, any of the red light irradiation step S₁ and the blue light irradiation step S₂ may be conducted earlier as desired in each irradiation cycle.

### (1-2) Plant cultivation method according to second embodiment

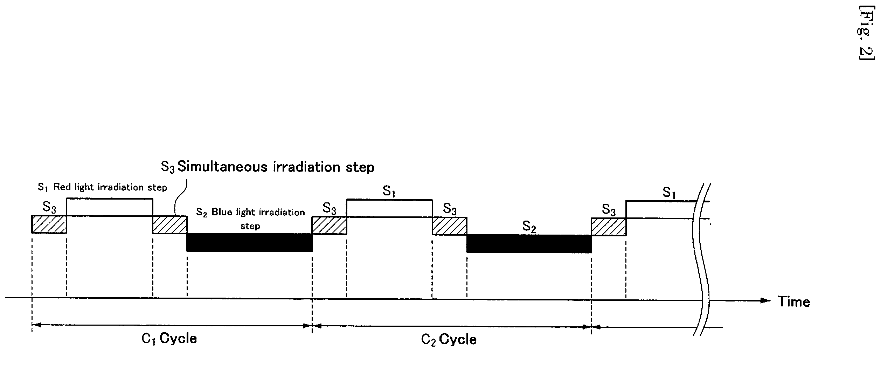

Fig. 2 is a schematic view illustrating the procedure of the plant cultivation method according to the second embodiment of the present invention. This embodiment conducts the red light irradiation step and the blue light irradiation step intermittently and repeatedly by allowing the both steps to be intervened by a step for irradiating the red illuminative light and the blue illuminative light simultaneously to the plant (hereinafter referred to also as "simultaneous irradiation step").

In the figure, the symbol S₃ represents the simultaneous irradiation step. In this embodiment, the red light irradiation step S₁ and the blue light irradiation step S₂ are conducted intermittently while being intervened by the simultaneous irradiation step S₃, and the irradiation cycle consisting of the red light irradiation step S₁, simultaneous irradiation step S₃, and blue light irradiation step S₂ is conducted repeatedly.

While the case in which the procedure is started from the simultaneous irradiation step S₃ in the first irradiation cycle C₁ is exemplified here, any of the red light irradiation step S₁, simultaneous irradiation step S₃, and blue light irradiation step S₂ may be conducted earlier as desired in each irradiation cycle.

### (1-3) Plant cultivation method according to third embodiment

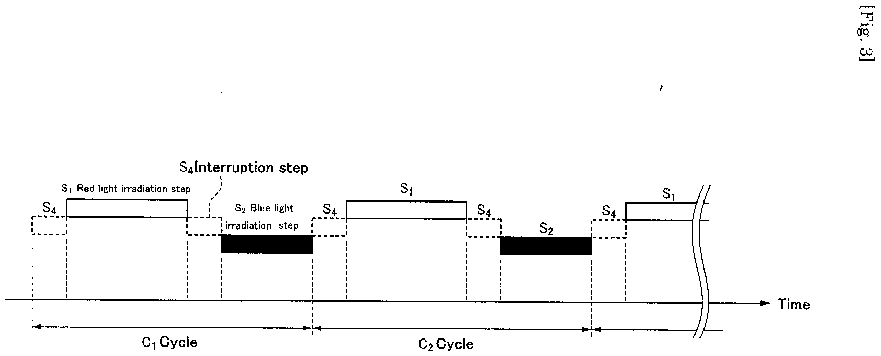

Fig. 3 is a schematic view illustrating the procedure of the plant cultivation method according to the third embodiment of the present invention. This embodiment conducts the red light irradiation step and the blue light irradiation step intermittently and repeatedly by allowing the both steps to be intervened by a step for interrupting the irradiation of the light to the plant (hereinafter referred to also as "interruption step").

In the figure, the symbol S₄ represents the interruption step. In this embodiment, the red light irradiation step S₁ and the blue light irradiation step S₂ are conducted intermittently while being intervened by the interruption step S₄, and the irradiation cycle consisting of the red light irradiation step S₁, the interruption step S₄, and the blue light irradiation step S₂ is conducted repeatedly.

While the case in which the procedure is started from the interruption step S₄ in the first irradiation cycle C₁ is exemplified here, any of the red light irradiation step S₁, interruption step S₄, and blue light irradiation step S₂ may be conducted earlier as desired in each irradiation cycle.

### (1-4) Wavelength

In the plant cultivation method according to each embodiment described above, the red light is a light having a peak wavelength of 570 to 730 nm, and a light having a peak wavelength of 635 to 660 nm is employed preferably. On the other hand, the blue light is a light having a peak wavelength of 400 to 515 nm, and a light having a peak wavelength of 400 to 460 nm is employed preferably.

The wavelength of the red light and the blue light may vary within the aforementioned wavelength range, and it is possible to change the wavelength for example in the Nth (N is an integer of 1 or more) irradiation cycle C_{N}. It is also possible that the wavelength may be different between the Nth irradiation cycle C_{N} and the Mth (M is an integer of 1 or more which is different from N) irradiation cycle C_{M} within the aforementioned wavelength range.

Furthermore, it is also possible that, in the aforementioned red light irradiation step S₁, simultaneous irradiation step S₃, and blue light irradiation step S₂, the red light and the blue light may be combined with lights having other wavelength ranges to conduct an irradiation with the lights having several wavelength ranges.

### (1-5) Light quantity(intensity)

While the light quantities (intensities) of the red illuminative light and the blue illuminative light in the red light irradiation step S₁, the blue light irradiation step S₂ and the simultaneous irradiation step S₃ are not limited particularly, each, when expressed for example as a photosynthetic photon flux density (PPFD), is approximately 1 to 1000 µmol/m²s, preferably 10 to 500 µmol/m²s, more preferably 20 to 250 µmol/m²s.

While the light quantity (intensity) ratio of the red illuminative light and the blue illuminative light in the aforementioned each step may be set as desired, a ratio of "red:blue" or "blue:red" within the range of about 1:1 to 20:1 is preferred. Typically, the light quantity ratio as "red:blue" or "blue:red" can be set for example at 1:1, 5:3, 2:1, 3:1, 4:1, 10:1, 20:1, and the like. The light quantity ratio as "red:blue" is 1:1 to 3:1 in an especially preferred case.

The light quantity of the red light and the blue light may vary within the aforementioned range, and it is possible to change the light quantity for example in the Nth (N is an integer of 1 or more) irradiation cycle C_{N}. It is also possible that the light quantity may be differentiated between the Nth irradiation cycle C_{N} and the Mth (M is an integer of 1 or more which is different from N) irradiation cycle C_{M} within the aforementioned range.

### (1-6) Irradiation time

In the plant cultivation method according to aforementioned each embodiment, the time period of a single irradiation cycle is the entire cultivation period at maximum. The shortest period may be set as desired as long as the effect of the invention can be exerted. The time period of a single irradiation cycle may employ, for example an hour (h) as a time length unit, or may employ a longer time length unit (for example, day (D)) or a shorter time length unit (for example, minute (min)).

For example, in the plant cultivation method according to the first embodiment wherein the red light irradiation step S₁ and the blue light irradiation step S₂ are conducted alternately and successively, if the single irradiation cycle takes a single day, then the red light irradiation step S₁ may take 12 hours and the blue light irradiation step S₂ may take 12 hours. Also for example, if the irradiation cycle is repeated 4 times a day, then the single irradiation cycle takes 6 hours, and the red light irradiation step S₁ may take 3 hours and the blue light irradiation step S₂ may take 3 hours.

The time period of a single irradiation cycle may vary between the Nth irradiation cycle C_{N} and the Mth (M is an integer of 1 or more which is different from N) irradiation cycle C_{M}. For example, the irradiation cycle C_{N} may take 12 hours and the subsequent irradiation cycle C_{N+1} may take 6 hours.

The ratio of the time periods of the red light irradiation step S₁, blue light irradiation step S₂, simultaneous irradiation step S₃, and interruption step S₄ within a single irradiation cycle may be set as desired. In the plant cultivation method according, for example, to the aforementioned first embodiment, if a single irradiation cycle takes a single day, then "the red light irradiation step S₁/the blue light irradiation step S₂" may be set as desired for example to "12 hours/12 hours (1:1)", "16 hours/8 hours (2:1)", "21 hours/3 hours (7:1)", and the like.

Most preferably, in the plant cultivation method according to the first embodiment where the red light irradiation step S₁ and the blue light irradiation step S₂ are conducted alternately and successively, the irradiation time of the red light irradiation step S₁ and the blue light irradiation step S₂ is 0.1 hour or longer and less than 48 hours. For the purpose of obtaining a high plant growth promoting effect, the irradiation time of the red light irradiation step S₁ and the blue light irradiation step S₂ is 3 hours or longer and 24 hours or less in the most preferred case. In such a case, the ratio of the time periods of the red light irradiation step S₁ and the blue light irradiation step S₂ may be selected as desired, and "the red light irradiation step S₁/the blue light irradiation step S2" may be "18-hours/6-hours".

### (2) Condition establishing step

The plant cultivation method according to the present invention preferably includes a step for establishing the irradiation conditions of the red illuminative light and the blue illuminative light on the previous stage of the aforementioned cultivation step. In such a condition establishing step, the irradiation conditions of the red illuminative light and the blue illuminative light capable of giving, in the illumination environment employing the illuminative light comprising the red illuminative light and the blue illuminative light, a growing effect equivalent or superior to that in the white light illumination environment on the plant as a cultivation target is established. By conducting the alternate irradiation of the red illuminative light and the blue illuminative light in the cultivation step in accordance with the irradiation conditions thus established, the growth promoting effect can more surely be obtained. It is also possible to obtain the growth promoting effect by conducting only the cultivation step while omitting the condition establishing step.

In this step, a plant is cultivated first in a white light illumination environment and the growth of the plant is recorded. The white light employed here may also be a natural light. Thereafter, the plant is cultivated in an illumination environment where the red illuminative light and the blue illuminative light are irradiated simultaneously. Here, the irradiation conditions of the red illuminative light and the blue illuminative light are provided in many ways, among which the irradiation conditions capable of giving a growing effect equivalent or superior to that in the white light illumination environment recorded previously are searched for. As the irradiation conditions, the light quantity ratio between the red illuminative light and the blue illuminative light, the total light quantity, the wavelength, and the like should be investigated. For the growth in the white light illumination environment, a reference may be made not only to the actual test data but also to known data from documents, and the like.

This step is conducted typically in the manner for example as shown below. First, the plant is cultivated in a fluorescent light illumination environment at a light quantity (PPFD) of 140 µmol/m²s. Next, several conditions of total light quantities are established within the range of about 100 to 500 µmol/m²s, and combined with several conditions of the light quantity ratio as "red:blue" or "blue:red" of about 1:1 to 20:1, and the plant is cultivated in the simultaneous irradiation environment. Then, the total light quantity and the light quantity ratio which gave the growing effect equivalent or superior to that in the fluorescent light illumination environment are specified.

The plant cultivation method according to the present invention is considered to exert a remarkable plant growth promoting effect by allowing the irradiation with the red light and the blue light to act in harmony with the photosynthesis mechanism of the plant. In the plant cultivation method according to the present invention, the plant growth promoting effect can further be enhanced when combined with the use of carbon dioxide gas or known agents having growth promoting effects.

### 2. Plant cultivation equipment

### (1) Plant cultivation equipment according to first embodiment

### (1-1) Light irradiation part

The plant cultivation equipment according to the first embodiment can perform each procedure of the aforementioned plant cultivation method, and comprises a light irradiation part for irradiating a red illuminative light and a blue illuminative light to the plant; and a controlling part for controlling the light irradiation part to conduct a step for irradiating the red illuminative light to the plant and a step for irradiating the blue illuminative light to the plant separately and independently of each other within a certain time period.

The light irradiation part comprises light sources which emit the red light and the blue light. The light sources of the red light and the blue light may be known light sources, which can be employed independently or in combination. The light source of the red illuminative light is preferably a light source which emits a light containing the red light and no blue light, more preferably a light source which emits only the red light. Also the light source of the blue illuminative light is preferably a light source which emits a light containing the blue light and no red light, more preferably a light source which emits only the blue light. Nevertheless, as a light source of the blue illuminative light, a light source containing a blue light as a wavelength component such as a fluorescent lamp may also be employed in some cases, and, as a light source of the red illuminative light, a light source containing a wavelength component other than the red light may also be employed in some cases.

A light source which is employed preferably is a photosemiconductor device such as a light emitting diode (LED) or a laser diode (LD) which allows the wavelength to be selected easily and emits a light having a high proportion of the photo energy of the valid wavelength range. When using an electroluminescence (EL), the EL may be organic or inorganic.

The photosemiconductor device is compact-sized, long-lived, and capable of emitting at a specific wavelength depending on the material with no unnecessary heat emission thereby achieving a favorable energy efficiency, and hardly gives any impairment such as leaf scorch even with the irradiation from a close proximity. As a result, by using the photosemiconductor device as a light source, it becomes possible to conduct the cultivation at a lower electric power cost in a smaller space when compared with other light source.

The light source may be an SMD line light source in which SMDs each having a combination of a single red photosemiconductor device and a single blue photosemiconductor device mounted thereon (2 Chips Surface Mount Device) are aligned linearly, or a single color line light source or a single color panel light source in which only either one of the red photosemiconductor devices or the blue photosemiconductor devices are aligned linearly or planarly.

The semiconductor device is capable, in principle, of flicker operation at a frequency as high as several megahertz (MHz) or higher. Accordingly, by using the photosemiconductor device as a light source, the red light irradiation step S₁, the blue light irradiation step S₂, the simultaneous irradiation step S₃, and the interruption step S₄ can be switched to each other very quickly.

As LEDs emitting lights having the aforementioned wavelength ranges, a red LED such as an aluminum/gallium/indium/phosphorus-based light emitting diode (gallium/phosphorus-based board, red wavelength: 660 nm) marketed under a product number of HRP-350F by Showa Denko K.K. and a blue LED such as a light emitting diode having a product number of GM2LR450G of the aforementioned company are exemplified.

The light sources of the light emitting diodes may for example be tubular and compact fluorescent lamps, and bulbar fluorescent lamps, high intensity discharge lamps, metal halide lamps, laser diodes, and the like. In combination with these light sources, an optical filter for selective use of the light in the aforementioned wavelength range may be employed.

### (1-2) Controlling part

The controlling part allows the light quantity (intensity), wavelength, and/or irradiation time of the red illuminative light and the blue illuminative light irradiated from the light irradiation part to be kept at certain values or to be varied in certain patterns.

The controlling part can be constructed using a versatile computer. When using, for example, an LED as a light source, the controlling part serves, based on the controlling pattern stored or memorized preliminarily in a memory or a hard disc, to adjust the level of the LED operation current and alter the light quantity ratio, total light quantity and the irradiation time of the red illuminative light and the blue illuminative light. In addition, the controlling part serves, based on the controlling pattern, to operate several LEDs emitting the lights in different wavelength ranges while switching them to each other, thereby altering the wavelength range of the irradiated light.

### (2) Plant cultivation equipment according to second embodiment

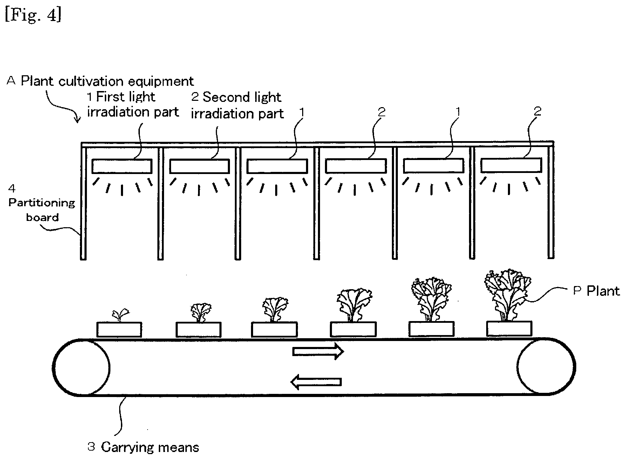

Fig. 4 shows a schematic view of the construction of the plant cultivation equipment according to the second embodiment. In the figure, the plant cultivation equipment indicated by the symbol A is provided with first light irradiation parts 1 which irradiate red illuminative lights and second light irradiation parts 2 which irradiate blue illuminative lights. The plant cultivation equipment A is provided also with a carrying means 3 for moving Plant P between positions irradiated with the illuminative lights originated from the first light irradiation parts 1 and positions irradiated with the illuminative lights originated from the second light irradiation parts 2. The figure exemplifies a case constructed by using single color panel light sources as the first light irradiation parts 1 and the second light irradiation parts 2 and using a conveyer as a carrying means 3 on which Plant P can be mounted (in figure, the block arrows indicates the conveyer operation directions).

The plant cultivation equipment A is constructed in such a manner that the aforementioned plant cultivation method according to the first embodiment can be practiced, and the first light irradiation part 1 and the second light irradiation part 2 are provided as being intervened by a partitioning board 4, and aligned alternately along with the direction in which Plant P is moved by the carrying means 3. The first light irradiation part 1 and the second light irradiation part 2 are provided as two pairs or more. Since Plant P carried to the position beneath the first light irradiation part 1 is protected by the partitioning boards 4 from the blue illuminative light emitted from the adjacent second light irradiation part 2, it is irradiated only with the red illuminative light from the first light irradiation part 1. Similarly, Plant P carried to the position beneath the second light irradiation part 2 is irradiated only with the blue illuminative light.

In the plant cultivation equipment A, the carrying means 3 moves Plant P in a single direction beneath the alternately aligned first light irradiation parts 1 and second light irradiation parts 2 and Plant P is irradiated with the red illuminative light and the blue illuminative light alternately, thereby promoting the growth of Plant P. It is also possible to suppress the succulent growth to increase the crop.

In the plant cultivation equipment A, it is preferable to operate the carrying means 3 so that Plant P is moved from the position irradiated with the illuminative light originated from the earliest first light irradiation part 1 through the position irradiated with the illuminative light originated from the latest second light irradiation part 2 over the entire cultivation period of Plant P. The numbers of the first light irradiation part 1 and the second light irradiation part 2 to be provided and the operation speed of the carrying means 3 (thus Plant P moving speed) may be established as appropriate depending on the cultivation period of Plant P, the irradiation cycle (see Fig. 1, symbol C₁) period, and the like. For example, when the cultivation period is 30 days and the irradiation cycle for the red light irradiation step (also see symbol S₁) is 12 hours and that for the blue light irradiation step (also see symbol S₂) is 12 hours, each 30 first light irradiation parts 1 and second light irradiation parts 2 are provided, and the carrying means 3 is operated at a speed allowing Plant P to be positioned beneath each light irradiation parts for each 12 hours.

The interval at which the partitioning boards 4 are provided is also established as appropriate depending on the irradiation cycle time, and the like. For example, when a single irradiation cycle includes the red light irradiation step for 18 hours and the blue light irradiation step for 6 hours, then the distance between the two partitioning boards 4 constructing the first light irradiation part 1 is established to be 3 times the distance between the two partitioning boards 4 constructing the second light irradiation part 2. Also for example, when the time of the red light irradiation step in a single irradiation cycle is altered from the time of the red light irradiation step in the irradiation cycle at the previous stage, then the distance between the partitioning boards 4 constructing the single first light irradiation part 1 is established to be longer or shorter than the distance between the partitioning boards 4 constructing the first light irradiation part 1 at the previous stage correspondingly to the alteration in the time period.

Although the case is exemplified here in which Plant P is moved by the carrying means 3 in a single direction beneath the first light irradiation parts 1 and the second light irradiation parts 2 aligned alternately along with the Plant P movement direction, it is also possible in the plant cultivation equipment that the plant is allowed to be moved back and forth between a position irradiated with the red illuminative light originated from the first light irradiation part and a position irradiated with the blue illuminative light originated from the second light irradiation part. In such a case, at least one pair of the first light irradiation part and the second light irradiation part may be provided and the carrying means may serve to allow the plant to go back and forth beneath the two light irradiation parts.

The aforementioned plant cultivation equipment according to the second embodiment can be applied also for the purpose of practicing the plant cultivation method according to the aforementioned second embodiment and third embodiment. When applying to the plant cultivation method according to the second embodiment, a third light irradiation part which irradiates the red illuminative light and the blue illuminative light may be provided in the plant cultivation equipment A between the first light irradiation part 1 and the second light irradiation part 2. Alternatively, in the plant cultivation equipment A, by allowing the protection from lights by the partitioning board 4 to be imperfect partly, Plant P moving from the position beneath the first light irradiation part 1 to the position beneath the second light irradiation part 2 is allowed to be irradiated transiently with the red illuminative light and the blue illuminative light at the same time.

When applying to the plant cultivation method according to the third embodiment, the first light irradiation part 1 and the second light irradiation part 2 in the plant cultivation equipment A are intervened by a space where no light irradiation part is provided, and Plant P is allowed to move in a single direction beneath the first light irradiation part, the second light irradiation part and the aforementioned space.

### 3. Cultivated plants

The cultivated plants targeted by the plant cultivation method according to the present invention are not limited particularly, and may be vegetables, potatoes, mushrooms, fruits, legumes, cereals, nuts and seeds, ornamental plants, ferns, mosses, and the like. Also, the types of the cultivation of these plants are not limited particularly and may for example be hydroponics, soil cultivation, nutrient liquid cultivation, solid culture medium cultivation, and the like.

### (1) Leaf vegetables

The leaf vegetables may for example be plants of Brassicaceae such as potherb mustard, komatsuna Turnip leaf, karashimizuna leaf, leaf mustard, wasabina leaf, watercress, Chinese cabbage, tsukena leaf and analogues, green pak choi, cabbage, cauliflower, broccoli, Brussels sprouts, rocket, pino green, and the like; plants of Compositae such as lettuce and analogues, Boston lettuce, garland chrysanthemum, butterbur, Rororossa, red romaine, chicory, and the like; plants of Liliaceae such as onion, garlic, shallot, Chinese chive, asparagus, and the like, plants of Apiaceae such as parsley, Italian parsley, Japanese honeywort, celery, dropwort, and the like; plants of Labiatae such as beefsteak plant, basil, and the like; plants of Alliaceae such as leek; plants of Araliaceae such as udo; plants of Zingiberaceae; Japanese ginger, and the like.

Lettuce and analogues include head-forming lettuces, non-head-forming lettuces, and semi-head-forming lettuces, such as leaf lettuce, frilly lettuce, romaine, green wave, green leaf, red leaf, Frill-Ice (registered trademark), River Green (registered trademark), frill leaf, fringe green, No-chip lettuce, "MOKO" lettuce, Korean lettuce, Chima/Korean lettuce, and the like.

Fruiting vegetables may for example be plants of Cucurbitaceae such as melon, cucumber, squash, watermelon, and the like; plants of Leguminosae such as string bean, broad bean, pea, green soybean, and the like; plants of Solanaceae such as tomato, eggplant, green pepper, and the like; plants of Rosaceae such as strawberry and the like; plants of Malvaceae such as okra, plants of Poaceae such as maize and the like. Furthermore, root vegetables may for example be plants of Brassicaceae such as Japanese white radish, turnip, wasabi, and the like; plants of Compositae such as burdock and the like; plants of Apiaceae such as carrot and the like; plants of Solanaceae such as potato and the like; plants of Araceae such as taro and the like; plants of Convolvulaceae such as sweet potato and the like; plants of Dioscoreaceae such as yam and the like; plants of Zingiberaceae such as ginger plant and the like; plants of Nymphaeaceae such as lotus root and the like.

### (2) Fruits

Fruits may for example be fruits of Rosaceae such as raspberry, blackberry, boysenberry, downy cherry, and the like; fruits of Ericaceae such as blueberry, cranberry, and the like; fruits of Grossulariaceae such as gooseberry, red currant, and the like; fruits of Anacardiaceae such as mango ,and the like; fruits of Bromeliaceae such as pineapple and the like; fruits of Moraceae such as fig and the like; fruits of Vitaceae such as grape and the like; fruits of Caprifoliaceae such as blue honeysuckle and the like; fruits of Caricaceae such as papaya and the like; fruits of Passifloraceae such as passion fruit and the like; fruits of Cactaceae such as dragon fruit and the like.

### (3) Cereals

Cereals may for example be cereals of Poaceae such as foxtail millet, oats, barley, millet, wheat, rice, glutinous rice, maize, tear grass, barnyard grass, rye, and the like; cereals of Amaranthaceae such as grain amaranthus and the like; cereals of Polygonaceae such as buckwheat and the like.

### (4) Mosses and others

Mosses may for example be mosses belonging to Bryopsida. For example, those exemplified are mosses of genus Racomitrium in Grimmiaceae in Grimmiales, so-called Racomitrium moss, such as Racomitrium japonicum.

Ornamental plants targeted for the cultivation are rose, miniature rose, Japanese gentian as well as various foliage plants including ferns such as maidenhair, brake fern, little club moss, and the like.

### Examples

### <Test Example 1>

An example of plant cultivation method was subjected to Test Groups 1 to 8 whose light environments during growth were different from each other, which were then compared with each other to verify the correlation between the artificial light irradiation pattern and the growth promoting effect on the plants.

### A. Materials and methods

### (Materials)

In this Test Example, the subject employed for observing the growth state was a leaf lettuce (variety :Summer Surge). First, 6 seeds were seeded at regular intervals to a cultivation soil peatban, and allowed to germinate under a fluorescent lamp (12-hour daytime). During three days from seeding through germination, every Test Group was kept in an identical light environment. After germination, they were placed in respective artificial climate chambers whose light environments were different from each other, and allowed to grow for 21 days. The environments of the artificial climate chambers were all identical except for the light irradiation conditions, and set at a temperature of 25 to 27°C and a humidity of 50%.

### (Light sources)

The light sources employed for the light environments in this Test Example were three types of LED, namely, a red LED (center wavelength:660 nm, produced by Showa Denko K.K., HRP-350F), a red LED (center wavelength:635 nm, produced by Showa Denko K.K., HOD-350F), a blue LED (center wavelength:450 nm, produced by Showa Denko K.K., GM2LR450G), a white LED (near ultraviolet 405 nm excitation, produced by KYOCERA Corporation, TOP-V5000K) as well as fluorescent lamps. The number of mounts on a single set of each LED was 240 for both of 660 nm and 635 nm of the red LEDs and 240 for the blue LED, and 128 for the white LED.

Using each light source, the light environments of Test Groups 1 to 10 shown in Table 1 were prepared. Each light source's photosynthetic photon flux density (PPFD, µmolm⁻²s⁻¹) was adjusted at 140 µmolm⁻²s⁻¹ (except for Test Group 8 employing 80 µmolm⁻²s⁻¹ and Test Group 4 employing 160 µmolm⁻²s⁻¹) at the center of the cultivation soil peatban. When irradiating the lights at several wavelengths simultaneously or alternately, then the total of PPFDs of the respective irradiated lights was adjusted at 140 µmolm⁻²s⁻¹ (except for the Test Group 4 employing 160 µmolm⁻²s⁻¹). Table 1 shows the mean values of 5 points at the height near the surface of the soil contained in the cultivation soil peatban for the photosynthetic photon flux density (PPFD, µmolm⁻²s⁻¹), illuminance (lx), ultraviolet intensity (UV-A and UV-420, Wm⁻²), height from the light source (cm), and duty ratio (%) in the light environments of Test Groups 1 to 10. The details of the light environments, irradiated lights and irradiation patterns in the respective Test Groups are described below.

**[Table 1]**

| Test Group | Irradiated light | Irradiation time period (hours/day) | Interruption time period (hours/day) |
|---|---|---|---|
| 1 | 660 nm (red)/450 nm (blue), alternately | 24 (red 12/blue 12) | 0 |
| 2 | 660 nm (red)/450 nm (blue), simultaneously | 12 | 12 |
| 3 | 660 nm (red)/450 nm (blue), sim ultaneously | 24 | 0 |
| 4 | 635 nm (red)/450 nm (blue), alternately | 24 (red 12/blue 12) | 0 |
| 5 | 635 nm (red)/450 nm (blue), sim ultaneously | 12 | 12 |
| 6 | Only 660 nm (red) | 12 | 12 |
| 7 | Only 635 nm (red) | 12 | 12 |
| 8 | Only 450 nm (blue) | 12 | 12 |
| 9 | White light | 12 | 12 |
| 10 | Fluorescent lamp | 12 | 12 |

### (Test Group 1)

In this Test Group, a lettuce was irradiated for each 12 hours alternately with a red light (660 nm) and a blue light (450 nm). In this Test Group, the period during which no light was irradiated was not provided.

This Test Group employed a light environment involving a red light (660 nm) had an average PPFD of 80.7 µmolm⁻²s⁻¹, an average illuminance of 1000 lx, average ultraviolet intensity for both of UV-A and UV-420 was 0 Wm⁻², average height from the light source was 30 cm, and average duty ratio was 20%. The blue light (450 nm) had an average PPFD of 56.4 µmolm⁻²s⁻¹, an average illuminance of 182 lx, an average ultraviolet intensity for UV-A of 0 Wm⁻², and that for UV- 420 of 9.22 Wm⁻², an average height from the light source of 15 cm, and an average duty ratio of 30%.

### (Test Group 2)

In this Test Group, a lettuce was irradiated for 12 hours simultaneously with a red light (660 nm) and a blue light (450 nm), followed by 12 hours during which no light was irradiated, and this sequence was repeated.

This Test Group's PPFD, illuminance, ultraviolet intensity (UV-A and UV-420), height from the light source, and duty ratio were analogous to those in Test Group 1.

### (Test Group 3)

In this Test Group, a lettuce was irradiated for 24 hours simultaneously with a red light (660 nm) and a blue light (450 nm). In this Test Group, the period during which no light was irradiated was not provided.

This Test Group employed a light environment involving, as a total of a red light (660 nm) and a blue light (450 nm), an average PPFD of 145.3 µmolm⁻²s⁻¹, an average illuminance of 1184 lx, an average ultraviolet intensity for UV-A of 0 Wm⁻², and that for UV-420 of 9.05 Wm⁻², an average height from the light source for the red light (660 nm) of 30 cm, that for the blue light (450 nm) of 15 cm, an average duty ratio for the red light (660 nm) of 20%, and that for the blue light (450 nm) of 60%.

### (Test Group 4)

In this Test Group, a lettuce was irradiated for each 12 hours alternately with a red light (635 nm) and a blue light (450 nm). In this Test Group, the period during which no light was irradiated was not provided.

This Test Group's total PPFD of the red light (635 nm) and the blue light (450 nm), illuminance, ultraviolet intensity (UV-A and UV-420), height from the light source, and duty ratio were analogous to those in Test Group 1.

### (Test Group 5)

In this Test Group, a lettuce was irradiated for 12 hours simultaneously with a red light (635 nm) and a blue light (450 nm), followed by 12 hours during which no light was irradiated, and this sequence was repeated.

This Test Group's total PPFD of the red light (635 nm) and a blue light (450 nm), illuminance, ultraviolet intensity (UV-A and UV-420), height from the light source, and duty ratio were analogous to those in Test Group 1.

### (Test Group 6)

In this Test Group, a lettuce was irradiated for 12 hours only with a red light (660 nm), followed by 12 hours during which no light was irradiated, and this sequence was repeated.

This Test Group employed a light environment involving an average PPFD of 139.3 µmolm⁻²s⁻¹, an average illuminance of 1624 lx, an average ultraviolet intensity for both of UV-A and LTV-420 which was 0 Wm⁻², an average height from the light source of 30 cm, and an average duty ratio of 30%.

### (Test Group 7)

In this Test Group, a lettuce was irradiated for 12 hours only with a red light (635 nm), followed by 12 hours during which no light was irradiated, and this sequence was repeated.

This Test Group's PPFD of the red light (635 nm), illuminance, ultraviolet intensity (UV-A and UV-420), height from the light source, and duty ratio were analogous to those in Test Group 6.

### (Test Group 8)

In this Test Group, a lettuce was irradiated for 12 hours only with a blue light (450 nm), followed by 12 hours during which no light was irradiated, and this sequence was repeated.

This Test Group employed a light environment involving an average PPFD of 84.1 µmolm⁻²s⁻¹, an average illuminance of 283 lx, an average ultraviolet intensity for UV-A of 0.33 Wm⁻² and that for UV-420 of 14.5 Wm⁻², an average height from the light source of 15 cm, and an average duty ratio of 50%.

### (Test Group 9)

In this Test Group, a lettuce was irradiated for 12 hours only with a white light (405 nm excitation), followed by 12 hours during which no light was irradiated, and this sequence was repeated.

This Test Group employed a light environment involving an average PPFD of 142.0 µmolm⁻²s⁻¹, an average illuminance of 8204 lx, an average ultraviolet intensity for UV-A of 0.004 Wm⁻² and that for UV-420 of 3.74 Wm⁻², and an average height from the light source of 16 cm.

### (Test Group 10)

In this Test Group, a lettuce was irradiated for 12 hours only with a fluorescent lamp, followed by 12 hours during which no light was irradiated, and this sequence was repeated.

This Test Group employed a light environment involving an average PPFD of 139.8 µmolm⁻²s⁻¹, an average illuminance of 10680 lx, an average ultraviolet intensity for UV-A of 0.338 Wm⁻² and that for UV-420 of 4.11 Wm⁻², and an average height from the light source of 38 cm.

### B. Results

In the aforementioned Test Groups 1 to 10, the growing was started in different light environments after germination, and then the growth state was observed and measured at each time point after 7 days (10 days after seeding), after 14 days (17 days after seeding), and after 21 days (24 days after seeding) for comparison between Test Groups.

### (After 7-day growth)

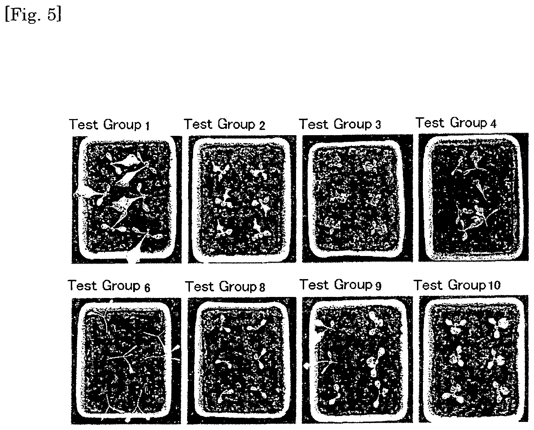

Fig. 5 shows photographs each representing the growth state in each Test Group 7 days after starting the growth in a different light environment. Also in Table 2, the stem length (mm), the first leaf length (cm), the number of true leaves (leaves) and the leaf width (cm) measured at the same time point in each Test Group are indicated. The measured values of the respective items indicate the 6 samples' "mean" or "minimum value - maximum value" seeded within an identical cultivation soil peatban.

**[Table 2]**

| Test Group | Irradiated light | Stem length (mm) | First leaf length (cm) | Number of true leaves (leaves) | Leaf width (cm) |
|---|---|---|---|---|---|
| 1 | 660 nm (red)/450 nm (blue), alternately | 0 - 5 | 5.3 - 6.5 | 2 | 2.3 - 2.5 |
| 2 | 660 nm (red)/450 nm (blue), simultaneously (12-hour irradiation) | 0 - 3 | 2.5 - 3.0 | 1 - 2 | - |
| 3 | 660 nm (red)/450 nm (blue), simultaneously (24-hour irradiation) | 1 - 2 | 2.0 - 2.9 | 1 - 2 | 1.5 - 2.0 |
| 4 | 635 nm (red)/450 nm (blue), alternately | - | - | - | - |
| 5 | 635 nm (red)/450 nm (blue), simultaneously | - | - | - | - |
| 6 | Only 660 nm (red) | 25 - 35 | 2.0 - 4.0 | 1 | 0.3 - 0.6 |
| 7 | Only 635 nm (red) | - | - | - | - |
| 8 | Only 450 nm (blue) | 2 - 4 | 1.9 - 2.0 | 1 | 1.1 - 1.4 |
| 9 | White light | 3 - 9 | 2.7 - 5.0 | 2 | 0.9 - 1.7 |
| 10 | Fluorescent lamp | 1 - 2 | 2.8 - 2.9 | 2 | 1.5 - 1.8 |

As shown in Fig. 5 and Table 2, the lettuce after 7-day growth under the alternate irradiation with the red light (660 nm) and the blue light (450 nm) in Test Group 1 had the first leaf length and the leaf width which were longer when compared with other Test Groups.

### (After 14-day growth)

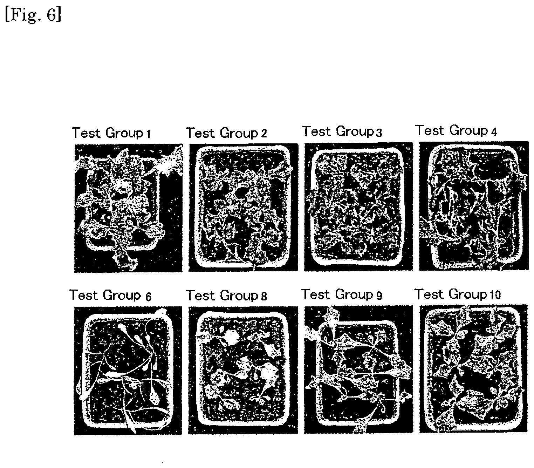

Fig. 6 shows photographs each representing the growth state in each Test Group 14 days after starting the growth in a different light environment. In each photograph, the size of the cultivation soil peatban is identical. Also in Table 3, the stem length (mm), the first leaf length (cm), the number of true leaves (leaves) and the leaf width (cm) measured at the same time point in each Test Group are indicated. The measured values of the respective items indicate, 6 samples' "mean" or "minimum value - maximum value" similarly to Table 2.

**[Table 3]**

| Test Group | Irradiated light | Stem length (mm) | First leaf length (cm) | Number of true leaves (leaves) | Leaf width (cm) |
|---|---|---|---|---|---|
| 1 | 660 nm (red)/450 nm (blue), alternately | 0 - 5 | 9.5 - 11.5 | 4 - 5 | 3.5 - 7.5 |
| 2 | 660 nm (red)/450 nm (blue), simultaneously (12-hour irradiation) | - | - | - | - |
| 3 | 660 nm (red)/450 nm (blue), simultaneously (24-hour irradiation) | 0 - 1 | 4.6 - 6.1 | 3 - 4 | 3.5 - 5.6 |
| 4 | 635 nm (red)/450 nm (blue), alternately | - | - | - | - |
| 5 | 635 nm (red)/450 nm (blue), simultaneously | - | - | - | - |
| 6 | Only 660 nm (red) | 40 - 55 | 8.7 - 9.5 | 2 | 1.5 - 1.8 |
| 7 | Only 635 nm (red) | - | - | - | - |
| 8 | Only 450 nm (blue) | 0 - 2 | 5.0 - 6.0 | 2 | 2.5 - 3.0 |
| 9 | White light | 4 - 13 | 8.0 - 8.5 | 3 - 4 | 3.3 - 4.2 |
| 10 | Fluorescent lamp | 0 - 2 | 5.0 - 6.5 | 4 | 3.5 - 3.8 |

As shown in Fig. 6 and Table 3, the lettuce after 14-day growth under the alternate irradiation with the red light (660 nm) and the blue light (450 nm) in Test Group 1 was characterized by the first leaf length which was longer when compared with other Test Groups. In addition, the number of the true leaves in Test Group 1 was greater by about 1 to 2 leaves when compared with other Test Groups.

### (After 21-day growth)

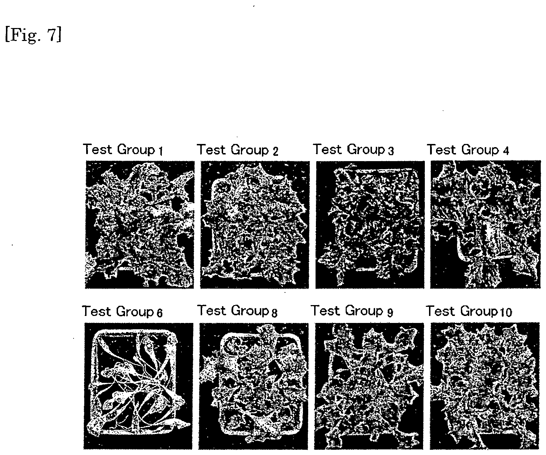

Fig. 7 shows photographs each representing the growth state in each Test Group 21 days after starting the growth in a different light environment. In each photograph, the size of the cultivation soil peatban is identical. Also in Table 4, the results of the comparison of the top fresh weight (g), the top dry weight (g), the number of true leaves (leaves), the stem length (cm), the leaf lamina length (cm), the leaf width (cm), and the leaf petiole length (cm) at the same time point in each Test Group are indicated while taking the growth in Test Group 10 (under fluorescent lamp) as 100%. The measured values of the respective items indicate the 6 samples' mean similarly to Table 2.

**[Table 4]**

| Test Group | Irradiated light | Top fresh weight (%) | Top dry weight (%) | Number of true leaves (%) | Stem length (%) | Leaf lamina length (%) | Leaf width (%) | Leaf petiole length (%) |
|---|---|---|---|---|---|---|---|---|
| 1 | 660 nm (red)/450 nm (blue), alternately | 252.8 | 286.7 | 97.4 | 108.3 | 139.4 | 142.4 | 79.0 |
| 2 | 660 nm (red)/450 nm (blue), simultaneously (12-hour irradiation) | 138.3 | 200.0 | 105.1 | 138.9 | 76.1 | 109.2 | 91.9 |
| 3 | 660 nm (red)/450 nm (blue), simultaneously (24-hour irradiation) | 134.9 | 228.7 | 92.3 | 52.8 | 72.0 | 103.0 | 39.5 |
| 4 | 635 nm (red)/450 nm (blue), alternately | 207.1 | - | - | - | 133.5 | 123.9 | 19.4 |
| 5 | 635 nm (red)/450 nm (blue), simultaneously | 129.0 | 153.3 | 105.1 | 145.8 | 73.6 | 106.4 | 120.2 |
| 6 | Only 660 nm (red) | 32.0 | 24.7 | 61.5 | 1190 | 76.5 | 40.8 | 378.2 |
| 7 | Only 635 nm (red) | 23.4 | 18.0 | 58.9 | 1514 | 53.9 | 37.0 | 498.4 |
| 8 | Only 450 nm (blue) | 62.8 | 66.7 | 64.2 | 73.6 | 75.5 | 80.1 | 96.0 |
| 9 | White light | 107.8 | 106.7 | 94.9 | 194.4 | 106.6 | 93.8 | 171.8 |
| 10 | Fluorescent lamp | 100.0 | 100.0 | 100.0 | 100.0 | 100.0 | 100.0 | 100.0 |

In Table 4, the lettuce under the alternate irradiation of the red light (660 nm) and the blue light (450 nm) in Test Group 1 had a top fresh weight greater by 2 times or more when compared with Test Group 10 (under fluorescent lamp). Similarly, the lettuce under the alternate irradiation of the red light (635 nm) and a blue light (450 nm) in Test Group 4 also had a top fresh weight greater by about 2 times when compared with Test Group 10. On the other hand, the lettuce irradiated simultaneously with the red light and the blue light in Test Groups 2 and 5 had top fresh weights greater when compared with Test Group 10, which was however less than that in Test Group 1 or Test Group 4 employing the alternate irradiation. The top fresh weight of the lettuce in Test Group 3 which was irradiated for 24 hours simultaneously with the red light (660 nm) and the blue light (450 nm) was similar to the weight in Test Group 2 whose irradiation time was a half. Based on these result, the alternate irradiation with the red light and the blue light was revealed to promote the plant growth.

The number of the true leaves in Test Group 1 at the time point after 21 days was, unlikely to that after 14-day growth, almost same to those in Test Group 2 and Test Group 10. This may be due to the plateau of the growth for increasing the number of the leaves in Test Group 1 achieved during 14 to 21 days of the growth.

In Test Group 1 and Test Group 4, the leaf lamina length and the leaf width were longer when compared with Test Group 10. Such a tendency was not observed in the lettuces under the simultaneous irradiation conditions of the red light and the blue light in Test Groups 2, 3 and 5. On the other hand, the stem length in Test Group 2 and Test Group 5 were longer when compared with Test Group 10. Based on these results, it was shown that the alternate irradiation with the red light and the blue light serves to promote the growth exclusively of the leaves while suppressing the succulent growth of the stem when compared with the simultaneous irradiation.

Table 5 shows the proportions (%) of the assimilative organ (g) and the non-assimilative organ (g) in the top fresh weight (g) and the top dry weight (g) based on each total weight and the proportion (%) of the dry weight (g) based on the fresh weight (g) in each Test Group 21 days after starting the growth in a different light environment.

**[Table 5]**

| Test Group | Irradiated light | Top fresh weight (g) | Assimilative organ (%) | Non-assimilativ e organ (%) | Top dry weight (g) | Assimilative organ (%) | Non-assimilative organ (%) | Dry weight/ fresh weight (%) |
|---|---|---|---|---|---|---|---|---|
| 1 | 660 nm (red)/450 nm (blue), alternately | 6.80 | 90.7 | 9.3 | 0.430 | 93.0 | 7.0 | 6.2 |
| 2 | 660 nm (red)/450 nm (blue), sim ultaneously (12-hour irradiation) | 3.72 | 85.8 | 14.8 | 0.300 | 80.0 | 20.0 | 8.1 |
| 3 | 660 nm (red)/450 nm (blue), simultaneously (24-Hour irradiation) | 3.63 | 90.9 | 9.1 | 0.343 | 93.9 | 6.1 | 9.7 |
| 4 | 635 nm (red)/450 nm (blue), Alternately | 5.57 | 87.4 | 12.6 | - | - | - | - |
| 5 | 635 nm (red)/450 nm (blue), sim ultaneously | 3.47 | 84.7 | 15.3 | 0.230 | 82.6 | 17.4 | 6.6 |
| 6 | Only 660 nm (red) | 0.87 | 59.8 | 40.2 | 0.037 | 67.6 | 32.4 | 4.2 |
| 7 | Only 635 nm (red) | 0.64 | 46.9 | 53.1 | 0.027 | 55.6 | 44.4 | 4.3 |
| 8 | Only 450 nm (blue) | 1.69 | 84.6 | 15.4 | 0.099 | 89.9 | 10.1 | 5.9 |
| 9 | White light | 2.90 | 84.5 | 15.2 | 0.160 | 87.5 | 12.5 | 5.4 |
| 10 | Fluorescent lamp | 2.69 | 87.4 | 12.6 | 0.150 | 93.3 | 6.70 | 5.8 |

The top fresh weight and the top dry weight in Test Group 1 exhibited a higher proportion of the assimilative organ when compared with Test Group 2. Test Group 4 exhibited a higher proportion of the assimilative organ for the top fresh weight when compared with Test Group 5. These results are in agreement with the results of the growth promotion of the leaf part in Test Group 1 and Test Group 4 shown in Table 4 and the results of the observation of the growth state in Fig. 7.

### C. Conclusion

Based on the results of this Test Example, the alternate irradiation with the red light and the blue light was revealed to promote the plant growth. While the aforementioned alternate irradiation promotes the growth of the leaves, it was shown that the succulent growth of the stem is not promoted. Such an effect was not reproduced by the irradiation simultaneously with the red light and the blue light or the irradiation only with either one of the both, and was proven to be obtained exclusively by the alternate irradiation with the red light and the blue light. Based on the results shown in this Test Example, the plant cultivation method was proven to be effective in promoting the plant growth.

### <Test Example 2>

The time period of a single irradiation cycle and the time ratio of the red light irradiation step and the blue light irradiation step within a single irradiation cycle were changed to further investigate the growth promoting effect of the alternate irradiation with the red light and the blue light according to the invention.

### A. Materials and methods

### (Materials)

The material employed was a leaf lettuce (variety :Summer Surge) which was allowed to germinate similarly to Test Example 1. The temperature and the humidity in the artificial climate chamber were set at an environment similar to that in Test Example 1.

### (Light sources)

The red LED (center wavelength:660 nm, produced by Showa Denko K.K., HRP-350F), the blue LED (center wavelength:450 nm, produced by Showa Denko K.K., GM2LR450G), and the fluorescent lamp employed in Test Example 1 were employed.

### B. Condition establishing step

First, the cultivation was conducted in a fluorescent lamp illumination environment at a light quantity (PPFD) of 140 µmol/m²s (Test Group 1). Then, the cultivation was conducted in the simultaneous irradiation environment with the red light and the blue light, and the irradiation conditions capable of giving an equivalent or higher growing effect when compared with the growth in a white light illumination environment were searched for. As the irradiation conditions, a total light quantity of 140 µmol/m²s and a "red:blue" light quantity ratio of 5:3 were established.

As shown in Table 6, at the time after growth for 21 days, Test Group 2 employing the simultaneous irradiation with the red light and the blue light for 12 hours followed by irradiation of no light for 12 hours exhibited a top fresh weight, a leaf lamina length, a leaf width, and an assimilative organ fresh weight which were equivalent or superior to those in Test Group 1. Also Test Group 3 employing the simultaneous irradiation with the red light and the blue light for 24 hours with no period during which no light was irradiated exhibited similar results. The measured values of the respective items in the table indicate the mean values of 6 samples seeded in an identical cultivation soil peatban.

### C. Cultivation step

Under the irradiation conditions involving a total light quantity of the red light and the blue light of 140 µmol/m²s and a light quantity ratio of 5:3, the cultivation was conducted in an alternate irradiation illumination environment.

Test Groups 5 to 7 and 10 employing the alternate irradiation with the red light and the blue light each for 3, 6, 12, and 24 hours exhibited, after 21-day growth, a marked growth promoting effect on a top fresh weight, a leaf lamina length, a leaf width, and an assimilative organ fresh weight when compared with Test Groups 1 to 3. Test Groups 8 and 9 involving the alternate irradiation with the red light and the blue light with "18-hours/6-hours" or "6-hours/18-hours" switching also exhibited a marked growth promoting effect.

Test Example 1 employing the alternate irradiation with the red light and the blue light each for 1 hour allowed the stem length to be elongated, resulting in succulent growth. While Test Example 11 employing the alternate irradiation with the red light and the blue light each for 48 hours exhibited some effect when compared with Test Group 1 employing the fluorescent lamp illumination environment, its effect relative to Test Groups 2 and 3 in the simultaneous irradiation environment with the red light and the blue light was insufficient.

**[Table 6]**

| | 1 | 2 | 3 | 4 | 5 | 6 | 7 | 8 | 9 | 10 | 11 |
|---|---|---|---|---|---|---|---|---|---|---|---|
| Test Group | Fluorescent lamp | Red and blue simultaneous irradiation 12h | Red and blue simultaneous irradiation 24h | Red and blue alternate irradiation 1h/1h | Red and blue alternate irradiation 3h/3h | Red and blue alternate irradiation 6h/6h | Red and blue alternate irradiation 12h/12h | Red and blue alternate irradiation 18h/6h | Red and blue alternate irradiation 6h/18h | Red and blue alternate irradiation 24h/24h | Red and blue alternate irradiation 48h/48h |
| Top fresh weight (g) | 2.78 | 3.72 | 3.63 | 4.09 | 6.25 | 4.18 | 6.80 | 6.95 | 6.37 | 4.73 | 3.26 |
| Number of true leaves (leaves) | 6.5 | 6.8 | 6.0 | 5.3 | 5.0 | 5.0 | 6.3 | 6.0 | 5.2 | 4.7 | 4.8 |
| Stem length (cm) | 0.72 | 0.55 | 0.38 | 1.12 | 0.55 | 0.50 | 0.78 | 1.12 | 1.23 | 0.85 | 0.87 |
| Leaf lamina length (cm) | 8.74 | 7.36 | 6.96 | 12.04 | 12.77 | 10.62 | 13.48 | 13.76 | 13.75 | 11.47 | 10.01 |
| Leaf width (cm) | 6.56 | 7.03 | 6.63 | 8.02 | 10.36 | 7.36 | 9.17 | 9.19 | 9.13 | 8.95 | 6.75 |
| Leaf petiole length (cm) | 1.23 | 1.14 | 0.49 | 1.28 | 1.09 | 1.76 | 0.98 | 1.18 | 1.65 | 1.19 | 1.57 |
| Fresh weight assimilative organ (g) | 2.43 | 3.19 | 3.30 | 3.61 | 5.70 | 3.60 | 6.17 | 6.21 | 5.55 | 4.22 | 2.83 |
| Fresh weight non-assimil ative organ (g) | 0.35 | 0.55 | 0.33 | 0.47 | 0.55 | 0.58 | 0.63 | 0.74 | 0.82 | 0.52 | 0.43 |

### <Test Example 3>

While changing the cultivated plant, the growth promoting effect of the alternate irradiation with the red light and the blue light was further investigated.

### A. Materials and methods

### (Materials)

In this Test Example, the subject employed for observing the growth state was a potherb mustard (variety :SHAKISARA). First, 5 to 10 seeds were seeded at regular intervals to a cultivation soil peatban and allowed to germinate under a fluorescent lamp (12-hour daytime). During 3 days from seeding through germination and 7 days after germination, every Test Group was kept in an identical light environment. After germination, they were placed in respective artificial climate chambers whose light environments were different from each other, and allowed to grow for 14 days. The environments of the artificial climate chambers were all identical except for the light irradiation conditions, and set at a temperature of 25 to 27°C and a humidity of 50%.

### (Light sources)

The red LED (center wavelength:660 nm, produced by Showa Denko K.K., HRP-350F), the blue LED (center wavelength:450 nm, produced by Showa Denko K.K., GM2LR450G), and the fluorescent lamp employed in Test Example 1 were employed.

### B. Condition establishing step

First, the cultivation was conducted in a fluorescent lamp illumination environment at a light quantity (PPFD) of 140 µmol/m²s (Test Group 1A). Then, the cultivation was conducted in the simultaneous irradiation environment with the red light and the blue light, and the irradiation conditions capable of giving an equivalent or higher growing effect when compared with the growth in a white light illumination environment were searched for. As the irradiation conditions, a total light quantity of 140 µmol/m²s and a "red:blue" light quantity ratio of 1:1 and 1:3 were established.

As shown in Table 7, at the time after growth for 7 days, Test Groups 2 and 3 employing the simultaneous irradiation with the red light and the blue light for 12 hours followed by irradiation of no light for 12 hours exhibited fresh weights which were equivalent or superior to those in Test Group 1. The measured values of the respective items in the table indicate the mean values of 6 samples seeded in an identical cultivation soil peatban.

**[Table 7]**

| Test Group | 1A | 2 | 3 |
|---|---|---|---|
| | Fluorescent lamp | Red and blue simultaneous irradiation 12h 1:1 | Red and blue simultaneous irradiation 12h 1:3 |
| Top fresh weight (g) | 4.59 | 4.23 | 4.53 |
| Number of true leaves (leaves) | 21.3 | 19.2 | 18.5 |
| Stem length (cm) | 1.52 | 1.88 | 1.23 |
| Leaf lamina length (cm) | 11.13 | 9.80 | 9.82 |
| Leaf width (cm) | 4.98 | 5.24 | 4.47 |
| Leaf petiole length (cm) | 5.56 | 5.06 | 5.02 |
| Fresh weight assimilative organ (g) | 3.05 | 2.85 | 3.02 |
| Fresh weight non-assimilative organ (g) | 1.54 | 1.37 | 1.51 |

### C. Cultivation step

Under the irradiation conditions involving a total light quantity of the red light and the blue light of 140 µmol/m²s and a light quantity ratio of 1:1 or 1:3, the cultivation was conducted in an alternate irradiation illumination environment. In addition, the cultivation was conducted again in the fluorescent lamp illumination environment at a light quantity (PPFD) of 140 µmol/m²s (Test Group 1B).

As shown in Table 8, Test Groups 4 and 5 employing the alternate irradiation with the red light and the blue light each for 12 hours exhibited, after 7-day growth, a marked growth promoting effect on the fresh weight when compared with Test Group 1B.

**[Table 8]**

| Test Group | 1B | 4 | 5 |
|---|---|---|---|
| | Fluorescent lamp | Red and blue alternate irradiation 12h/12h 1:1 | Red and blue alternate irradiation 12h/12h 1:3 |
| Top fresh weight (g) | 2.78 | 3.51 | 3.72 |
| Number of true leaves (leaves) | 15.0 | 11.5 | 12.7 |
| Stem length (cm) | 1.62 | 1.92 | 1.58 |
| Leaf lamina length (cm) | 10.03 | 10.02 | 10.40 |
| Leaf width (cm) | 4.25 | 3.98 | 4.52 |
| Leaf petiole length (cm) | 5.22 | 7.54 | 7.56 |
| Fresh weight assimilative organ | 1.82 | 1.86 | 2.05 |
| Fresh weight non- assimilative organ (g) | 0.96 | 1.65 | 1.67 |

### <Test Example 4>

While changing the cultivated plant, the growth promoting effect of the alternate irradiation with the red light and the blue light was further investigated.

### A. Materials and methods

### (Materials)

In this Test Example, the subject employed for observing the growth state was a leek sprout. First, 6 seeds were seeded at regular intervals to a cultivation soil peatban, and allowed to germinate under a fluorescent lamp (12-hour daytime). During three days from seeding through germination, every Test Group was kept in an identical light environment. After germination, they were placed in respective artificial climate chambers whose light environments were different from each other, and allowed to grow for 24 days. The environments of the artificial climate chambers were all identical except for the light irradiation conditions, and set at a temperature of 25 to 27°C and a humidity of 50%.

### (Light sources)

The red LED (center wavelength:660 nm, produced by Showa Denko K.K., HRP-350F), the blue LED (center wavelength:450 nm, produced by Showa Denko K.K., GM2LR450G), and the fluorescent lamp employed in Test Example 1 were employed.

### B. Condition establishing step

First, the cultivation was conducted in a fluorescent lamp illumination environment at a light quantity (PPFD) of 140 µmol/m²s (Test Group 1). Then, the cultivation was conducted in the simultaneous irradiation environment with the red light and the blue light, and the irradiation conditions capable of giving an equivalent or higher growing effect when compared with the growth in a white light illumination environment were searched for. As the irradiation conditions, a total light quantity of 140 µmol/m²s and a "red:blue" light quantity ratio of 5:3 were established.

As shown in Table 9, at the time after growth for 14 days, Test Group 2 employing the simultaneous irradiation with the red light and the blue light for 12 hours followed by irradiation of no light for 12 hours exhibited a fresh weight and a leaf length which were equivalent or superior to those in Test Group 1. The measured values of the respective items in the table indicate the mean values of 6 samples seeded in an identical cultivation soil peatban.

### C. Cultivation step

Under the irradiation condition involving a total light quantity of the red light and the blue light of 140µmol/m²s and a light quantity ratio of 5:3, the cultivation was conducted in an alternate irradiation illumination environment.

Test Group 3 employing the alternate irradiation with the red light and the blue light each for 12 hours exhibited, after 14-day growth, a marked growth promoting effect on the fresh weight and the leaf length when compared with Test Groups 1 and 2.

**[Table 9]**

| Test Group | 1 | 2 | 3 |
|---|---|---|---|
| | Fluorescent lamp | Red and blue simultaneous irradiation 12h | Red and blue alternate irradiation 12h/12h |
| Fresh weight (g/10 plants) | 0.313 | 0.318 | 0.425 |
| Leaf length (cm) | 9.17 | 8.27 | 11.40 |

### <Test Example 5>

In various cultivated plants, the growth promoting effect of the alternate irradiation with the red light and the blue light was verified.

### (Materials)

The materials employed included two types of leaf lettuces (variety :Red Fire and Black Rose) which were allowed to germinate similarly to Test Example 1. The temperature and the humidity of the artificial climate chamber were set to give an environment identical to that in Test Example 1.

The materials also employed were a radish (variety : Red Chime) and a turnip (variety: NATSU-HAKUREI). First, 6 seeds were seeded at regular intervals to a cultivation soil peatban, and allowed to germinate under a fluorescent lamp (12-hour daytime). During three days from seeding through germination, every Test Group was kept in an identical light environment. After germination, they were placed in respective artificial climate chambers whose light environments were different from each other, and allowed to grow for 24 days. The environments of the artificial climate chambers were all identical except for the light irradiation conditions, and set at a temperature of 25 to 27°C and a humidity of 50%.

### (Light sources)

The red LED (center wavelength:660 nm, produced by Showa Denko K.K., HRP-350F), the blue LED (center wavelength:450 nm, produced by Showa Denko K.K., GM2LR450G), and the fluorescent lamp employed in Test Example 1 were employed. The photosynthetic photon flux densities (PPFD, µmolm⁻²s⁻¹) of the red light and the blue light were adjusted at 87.5 and 52.5 µmolm⁻²s⁻¹, respectively, in the center of the cultivation soil peatban.

The results obtained in Red Fire, Black Rose, and NATSU-HAKUREI were shown in Tables 10 to 13, respectively. The measured values of the respective items in the table indicate the mean values of 6 samples seeded in an identical cultivation soil peatban.

In any of the leaf lettuces, Test Groups employing the alternate irradiation with the red light and the blue light exhibited, after 21-day growth, marked growth promoting effects on the top fresh weight, the leaf lamina length, the leaf width, and the assimilative organ fresh weight when compared with Test Group 1 employing a fluorescent lamp illumination environment (see Tables 10 and 11).

**[Table 10]**

| Test Group | 1 | 2 | 3 | 4 |
|---|---|---|---|---|
| | Fluorescent lamp | Red and blue alternate irradiation 12h/12h | Red and blue alternate irradiation 18h/6h | Red and blue alternate irradiation 6h/18h |
| Top fresh weight (g) | 2.82 | 3.74 | 4.14 | 4.50 |
| Number of true leaves (leaves) | 6.5 | 5.0 | 5.3 | 5.0 |
| Stem length (cm) | 0.53 | 1.13 | 1.57 | 1.62 |
| Leaf lamina length (cm) | 8.58 | 10.37 | 11.24 | 11.50 |
| Leaf width (cm) | 6.04 | 6.97 | 7.04 | 8.28 |
| Leaf petiole length (cm) | 1.10 | 1.49 | 1.61 | 1.99 |
| Fresh weight assimilative organ (g) | 2.41 | 3.17 | 3.47 | 3.71 |
| Fresh weight non -assimilative organ (g) | 0.41 | 0.57 | 0.66 | 0.79 |

**[Table 11]**

| Test Group | 1 | 2 | 3 |
|---|---|---|---|
| | Fluorescent lamp | Red and blue simultaneous irradiation 12h | Red and blue alternate irradiation 12h/12h |
| Top fresh weight (g) | 1.00 | 2.15 | 3.13 |
| Number of true leaves (leaves) | 5.0 | 5.0 | 5.0 |
| Stem length (cm) | 0.23 | 0.37 | 1.78 |
| Leaf lamina length (cm) | 5.52 | 6.43 | 9.49 |
| Leaf width (cm) | 4.53 | 6.12 | 6.72 |
| Leaf petiole length (cm) | 0.86 | 0.64 | 1.17 |
| Fresh weight assimilative organ | 0.87 | 1.93 | 2.70 |
| Fresh weight non-assimilative organ (g) | 0.14 | 0.22 | 0.42 |

**[Table 12]**

| Test Group | 1 | 2 | 3 |
|---|---|---|---|
| | Fluorescent lamp | Red and blue simultaneous irradiation 12h | Red and blue alternate irradiation 12h/12h |
| Underground fresh weight (g) | 2.52 | 1.51 | 2.59 |
| Top fresh weight (g) | 4.36 | 3.25 | 4.12 |
| Root length (cm) | 2.28 | 2.00 | 2.47 |
| Root diameter (cm) | 1.77 | 1.56 | 1.88 |
| Number of true leaves (leaves) | 5.0 | 4.3 | 4.5 |
| Leaf lamina length (cm) | 8.42 | 7.44 | 8.76 |
| Leaf width (cm) | 4.94 | 4.24 | 4.71 |
| Leaf petiole length (cm) | 4.04 | 3.19 | 5.52 |
| Assimilative organ fresh weight (g) | 2.88 | 2.03 | 2.47 |
| Non-assimilative organ fresh weight (g) | 1.48 | 1.22 | 1.65 |

**[Table 13]**

| Test Group | 1 | 2 |
|---|---|---|
| | Fluorescent lamp | Red and blue alternate irradiation 12h/12h |
| Underground fresh weight (g) | 0.36 | 2.64 |
| Top fresh weight (g) | 2.40 | 4.34 |
| Root length (cm) | 1.42 | 3.12 |
| Root diameter (cm) | 0.57 | 1.40 |
| Number of true leaves (leaves) | 8.5 | 6.2 |
| Leaf lamina length (cm) | 5.16 | 9.10 |
| Leaf width (cm) | 3.88 | 5.58 |
| Leaf petiole length (cm) | 3.03 | 6.59 |
| Assimilative organ fresh weight (g) | 1.46 | 2.50 |
| Non-assimilative organ fresh weight (g) | 0.95 | 1.85 |

The radish in Test Group 3 employing the alternate irradiation environment exhibited a marked growth promoting effect when compared with Test Group 1 employing the fluorescent lamp illumination environment and Test Group 2 employing the simultaneous irradiation environment (After 21-day growth). The growth promoting effect was identified in terms of the root length and diameter (see Table 12). Also in the turnip after 21-day growth in Test Group employing the alternate irradiation with the red light and the blue light, the growth was promoted in terms at least of the root length and diameter when compared with Test Group 1 employing the fluorescent lamp illumination, indicating a substantial effect on the underground fresh weight (see Table 13).

### Industrial Applicability

According to the plant cultivation method according to the present invention, the plant growth can be promoted by a convenient method thereby increasing the number of cropping and the quantity of crops per unit time period. As a result, the plant cultivation method according to the present invention can be employed preferably in artificial cultivation of leaf vegetables, fruits, and cereals.

### Reference Signs List

- A:: Plant cultivation equipment
- P:: Plant
- S₁:: Red light irradiation step
- S₂:: Blue light irradiation step
- S₃:: Simultaneous irradiation step
- S₄:: Interruption step
- C₁, C₂:: Cycle
- 1:: First light irradiation part
- 2:: Second light irradiation part
- 3:: Carrying means
- 4:: Partitioning board

## Claims

1. A plant cultivation method for conducting a step of irradiating a red illuminative light to a plant and a step of irradiating a blue illuminative light to the plant separately and independently of each other, wherein the irradiation time of the step of irradiating the red illuminative light and the step of irradiating the blue illuminative light is 0.1 hour or more and less than 48 hours, the steps are conducted alternately and successively, and the light quantity ratio of the red illuminative light and the blue illuminative light is 3:1 to more than 1:1, i.e. a higher quantity of red light than blue light, wherein the light quantity is the photosynthetic photon flux density expressed as µmol/m²s.

2. The plant cultivation method according to Claim 1, wherein the irradiation time is 3 hours or more and 24 hours or less.

3. The plant cultivation method according to Claims 1 or 2, wherein the plant is a leaf vegetable, a fruit, or cereal.

## Patentansprüche

1. Pflanzenkultivierungsverfahren zum getrennten und voneinander unabhängigen Durchführen eines Schrittes des Bestrahlens einer Pflanze mit einem roten Beleuchtungslicht und eines Schrittes des Bestrahlens der Pflanze mit einem blauen Beleuchtungslicht, wobei die Bestrahlungszeit des Schrittes des Bestrahlens mit rotem Beleuchtungslicht und des Schrittes des Bestrahlens mit blauem Beleuchtungslicht 0,1 Stunde oder mehr und weniger als 48 Stunden ist, die Schritte werden abwechselnd und nacheinander durchgeführt werden und das Lichtmengenverhältnis des roten und des blauen Beleuchtungslichts 3:1 bis mehr als 1:1 beträgt, d.h. die Lichtmenge des roten Lichts größer als die des blauen Lichts ist, wobei die Lichtmenge die als µmol/m²s ausgedrückte photosynthetische Photonenflussdichte ist.

2. Pflanzenanbauverfahren nach Anspruch 1, wobei die Bestrahlungszeit 3 Stunden oder mehr und 24 Stunden oder weniger beträgt.

3. Pflanzenanbauverfahren nach den Ansprüchen 1 oder 2, wobei die Pflanze ein Blattgemüse, eine Frucht oder Getreide ist.

## Revendications

1. Procédé de culture de plantes mettant en œuvre une étape d'irradiation d'une lumière d'éclairage rouge sur une plante et une étape d'irradiation d'une lumière d'éclairage bleue sur la plante séparément et indépendamment l'une de l'autre, dans lequel la durée d'irradiation de l'étape d'irradiation de la lumière d'éclairage rouge et de l'étape d'irradiation de la lumière d'éclairage bleue est de 0.1 heure ou plus et de moins de 48 heures, les étapes sont réalisées alternativement et successivement, et le rapport de quantité de lumière de la lumière d'éclairage rouge et de la lumière d'éclairage bleue est de 3:1 à plus de 1:1, i.e. une quantité de lumière rouge plus élevée que la lumière bleue, dans lequel la quantité de lumière est la densité de flux photonique photosynthétique exprimée en µmol/m²s.

2. Procédé de culture des plantes selon la revendication 1, dans lequel la durée d'irradiation est de 3 heures ou plus et de 24 heures ou moins.

3. Procédé de culture de plantes selon les revendications 1 ou 2, dans lequel la plante est un légume-feuille, un fruit ou une céréale.
